# EUROPEAN PATENT SPECIFICATION

(11) **EP 4 144 323 B1**
(45) Date of publication and mention of the grant of the patent: **11.12.2024**
(21) Application number: 21797253.8
(22) Date of filing: 05.02.2021
(51) Int. Cl.: A61C 8/02, A61C 8/00, A61F 2/28

(54) **DENTAL MEMBRANE SET**
DENTALMEMBRANSATZ
ENSEMBLE MEMBRANE DENTAIRE

(30) Priority: 29.04.2020 KR 20200001482 U
(43) Date of publication of application: 08.03.2023
(73) Proprietor: Megagen Implant Co., Ltd., Dalseong-gun, Daegu 42921 (KR)
(72) Inventor: PARK, Kwang Bum, Daegu 42016 (KR)
(74) Representative: Patentanwälte Hemmer Lindfeld Frese Partnerschaft mbB
(86) International application number: PCT/KR2021/001543
(87) International publication number: WO 2021/221277

(56) References cited:
- KR-A- 20120 101 265
- KR-A- 20130 083 204
- KR-A- 20140 017 368
- KR-A- 20160 091 195
- KR-A- 20160 092 794
- KR-B1- 101 405 839
- KR-B1- 101 741 255
- KR-B1- 101 741 255
- US-A1- 2001 012 607
- US-A1- 2012 135 376

## Description

### TECHNICAL FIELD

The present inventive concept relates to a dental membrane set which may prevent the gum epithelium from first entering a damaged portion and also guide bone regeneration, by adapting a bone defect portion where a bone defect occurs through an effective structure compared with the related art, and a dental membrane set comprising the same.

### BACKGROUND ART

An implant originally means a substitute used to restore lost human tissues. In a dental field, however, the implant refers to a kind of an instrument or procedure to implant an artificial tooth.

It is a procedure to restore the function of a tooth by placing a fixture on an alveolar bone where a tooth is lost, and then fixing an artificial tooth thereon. The fixture is a tooth root made of titanium and the like having no rejection by the human body to replace a lost tooth root (root).

When bone mass is insufficient or a lack of bone volume is expected, before implantation of a fixture, or a part of an implant is exposed outside the bone for various reasons (alveolar bone resorption due to inflammation, etc.), a procedure such as guided bone regeneration (GBR) is performed.

In many cases of GBR procedures, it has been known that a dental membrane and various bone materials (autogenous bone, allogeneic bone, xenogeneic bone, and synthetic bone) are used.

GBR is a procedure to form a space by using a membrane in an edentulous area regardless of periodontal tissue, and guide introduction of osteoblast into a bone defect portion in a lower portion of the space, thereby promoting bone tissue regeneration. It has been reported that the origin of GBR is developed from guided tissue regeneration (GTR).

A dental membrane used in performing GBR serves to prevent the gum epithelium that is fast regenerated, from growing and first entering a damaged portion. Also, it has been reported that the dental membrane has the ability to maintain adequate space and helps a lot in the differentiation of connective tissue and regeneration of bone.

The dental membrane is divided into an absorbent membrane and a non-absorbable membrane according to the physical properties thereof. Absorbent membranes include product names, such as Biogide, Biomend Extend, Ossix, Resolut, and the like, and non-absorbent membranes include expanded-polyetrafluoroethylene (e-PTFE), a titanium mesh, and the like.

A titanium mesh is a typical one of non-absorbent membranes, and actually titanium meshes have been widely used in dental treatment. The titanium mesh is manufactured in the form of a rectangular titanium plate with a plurality of holes perforated therein.

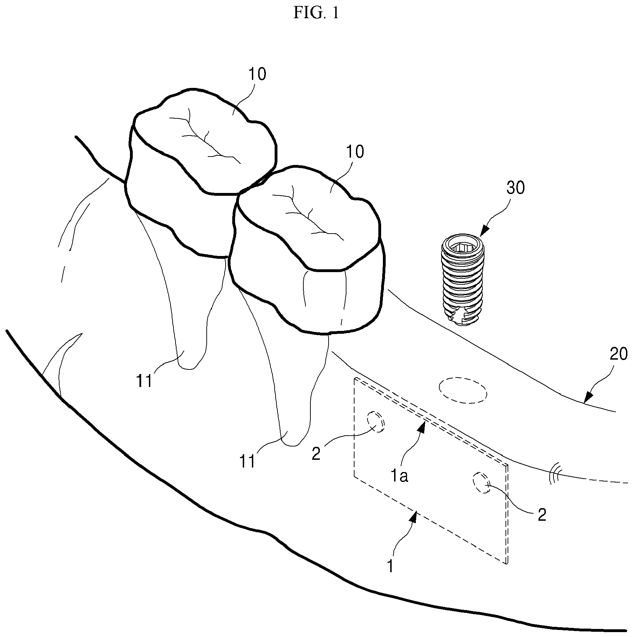

A process of treating a membrane as a titanium mesh or an implant surgery through the process are briefly described with reference to FIG. 1.

Referring to FIG. 1, a large number of teeth 10 are arranged on a gum 20. The teeth 10 are the primary means of digestion by breaking down food and sending the broken food to the stomach, and it may be different for each person, but there are usually about 28 teeth.

When one of the teeth 10 is lost (the posterior part is lost, etc.), due to the lost the tooth 10, not only does the aesthetics deteriorate, but it is inevitably very uncomfortable to chew food.

Accordingly, implantation is performed by placing a fixture 30, as a means to replace a tooth root 11 of the teeth 10, on the gum 20 of the lost tooth 10.

In this case, however, when the tooth root 11 is normal, the fixture 30 is placed and wrapped with a crown later. Otherwise, as described above, guided bone regeneration surgery is performed.

In this case, for example, a membrane 1 as a titanium mesh is fixed to a corresponding portion by using a screw 2 to prevent gum epithelium that is fast regenerated, from growing and first entering a damaged portion, and also help a lot in the differentiation of connective tissue and the regeneration of bone.

When performing a treatment on the membrane 1, first, an operator recognizes the accurate shape of a bone defect portion where a bone defect occurs, appropriately trims the membrane 1 into a desired shape, and then bends the membrane 1 three-dimensionally to adapt the bone defect portion.

Then, the membrane 1 is finally coupled to a fixing portion such as an alveolar bone and the like by using the screw 2.

The above method may be the most general method of the guided bone regeneration surgery. However, in this classical method that uses the membrane 1 by trimming and then bending the same, even when an end area 1a of the membrane 1 is bent, the end area 1a of the membrane 1 is not placed or pressed toward the side of an alveolar bone, but protrudes to the opposite side, that is, the side of the gum 20, or passes through the gum 20, thereby deteriorating the effect of treatment for bone regeneration.

US 2001/012607 A1 describes a method of growing a jaw bone and the related guided-tissue regeneration plate support. KR 2014 0017368 A describes a shielding membrane for alveolar bone regeneration which is located between a bone transplant material transplanted on the alveolar bone and soft tissues of the gum for alveolar bone regeneration. KR 101 741 255 B1 describes a membrane for regenerating alveolar bone having a thickness gradient. US 2012/135376 A1 describes a biocompatible, resorbable collagen membrane having a wedge shape.

### DETAILED DESCRIPTION OF THE INVENTIVE CONCEPT

### TECHNICAL PROBLEM

The invention relates to a dental membrane set according to claim 1. Provided is a dental membrane which may prevent the gum epithelium from first entering a damaged portion and also guide bone regeneration, by adapting a bone defect portion where a bone defect occurs through an effective structure compared with the related art, and furthermore reduce failure of guided bone regeneration (GBR), and a dental membrane set comprising the same.

### ADVANTAGEOUS EFFECTS

According to the present inventive concept, the gum epithelium may be prevented from first entering a damaged portion by adapting a bone defect portion where a bone defect occurs through an effective structure compared with the related art, bone regeneration may also be guided, and furthermore, the failure of guided bone regeneration (GBR) may be reduced.

### DESCRIPTION OF THE DRAWINGS

- FIG. 1: illustrates a typical membrane procedure process.
- FIG. 2: schematically illustrates a procedure state of a dental membrane set, according to a first embodiment of the present inventive concept.
- FIG. 3: is an enlarged perspective view of the dental membrane set of FIG. 2.
- FIG. 4: is a partially exploded view of FIG. 3.
- FIG. 5: is a side view of a dental membrane.
- FIG. 6: illustrates a state in which a large area end bending portion of FIG. 5 is bent.
- FIG. 7: is a perspective view of a dental membrane set according to a second embodiment of the present inventive concept.
- FIG. 8: is a cross-sectional view taken along line A-A of FIG. 7.
- FIG. 9: is a perspective view of a dental membrane set according to a third embodiment of the present inventive concept.

### BEST MODE

A dental membrane of the inventive dental membrane set includes a main plate portion preventing a gum epithelium from first entering a damaged portion and also guiding bone regeneration, by adapting a bone defect portion where a bone defect occurs, and a large area end bending portion forming an end area in one side of the main plate portion, manufactured as one body with the main plate portion, having a larger cross-sectional area than the main plate portion, and being bendable to one side with respect to the main plate portion.

The outer surface of the large area end bending portion may be processed to be round, and the large area end bending portion may be arranged in the bone defect portion to adapt a part of an uppersurface in a side surface corner of the bone defect portion.

The large area end bending portion may be manufactured to have a cross-sectional area that gradually increases from an area contacting the main plate portion toward an end portion thereof.

A plurality of through-holes may be formed in the large area end bending portion to cross a direction in which the large area end bending portion is bent.

The main plate portion may include a rectangular plate-type main plate portion.

The main plate portion may include a triangular convex-type main plate portion.

The triangular convex-type main plate portion may include triangular convex-type main plate portions continuously connected to each other in a lateral direction.

According to the present inventive concept, a dental membrane set includes a dental membrane preventing a gum epithelium from first entering a damaged portion and also guiding bone regeneration, by adapting a bone defect portion where a bone defect occurs, and a membrane fixing member fixing the dental membrane to the bone defect portion, wherein the dental membrane includes a main plate portion, and a large area end bending portion forming an end area in one side of the main plate portion, manufactured as one body with the main plate portion, having a larger cross-sectional area than the main plate portion, and being bendable to one side with respect to the main plate portion.

The membrane fixing member includes a bone screw having one side fixed to the bone defect portion, and a bone tack coupled to the bone screw with the dental membrane therebetween to fix the dental membrane.

The bone screw includes a screw portion fixed to the bone defect portion by a screw method, and a cuff formed coaxially with the screw portion, arranged around the screw portion, and forming a section having no screw thread.

An end portion of the screw portion may have a sharp tip portion, a non-screw portion having no screw thread may be formed between the sharp tip portion and the screw portion, and a tool insertion portion having a non-circular shape may be formed in an end portion of the cuff.

The bone tack may include a plurality of unit tacks formed by a cut portion, and a press-fit portion provided at a central portion of the plurality of unit tacks and press-fitted to the tool insertion portion, and the cut portion may be formed along the circumferential direction at equal intervals.

An outer surface of the large area end bending portion may be processed to be round, and the large area end bending portion may be arranged in the bone defect portion to adapt a part of an uppersurface in a side surface corner of the bone defect portion, and the large area end bending portion may be manufactured to have a cross-sectional area that gradually increases from an area contacting the main plate portion toward an end portion thereof.

A plurality of through-holes may be formed in the large area end bending portion to cross a direction in which the large area end bending portion is bent.

The main plate portion may include a rectangular plate-type main plate portion or a triangular convex-type main plate portion.

The triangular convex-type main plate portion may include triangular convex-type main plate portions continuously connected to each other in a lateral direction.

### MODE OF THE INVENTIVE CONCEPT

In order to fully understand the operational advantages of the present inventive concept and the objectives achieved by the implementation of the present inventive concept, the accompanying drawings illustrating preferred embodiments of the present inventive concept and the contents described in the accompanying drawings are referred to.

Hereinafter, the inventive concept will be described in detail by explaining preferred embodiments of the inventive concept with reference to the attached drawings. Like reference numerals in the drawings denote like elements.

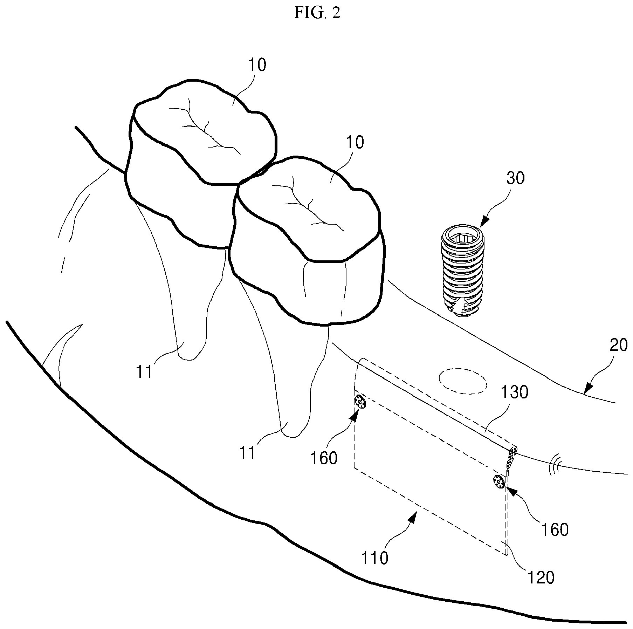

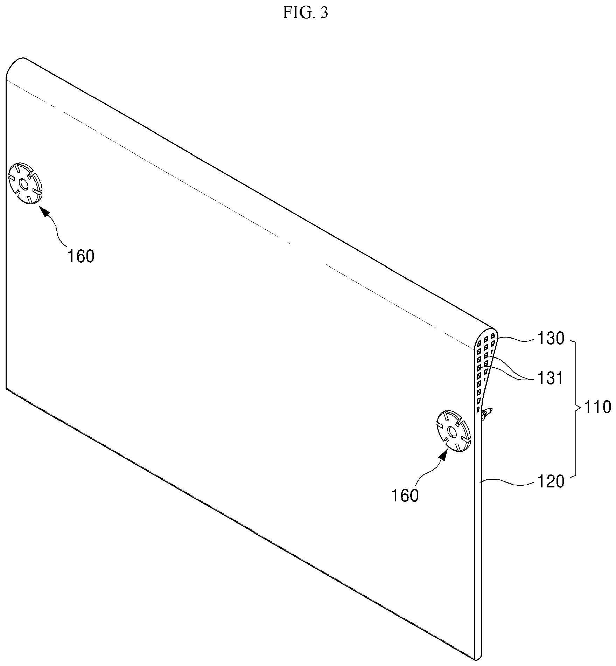

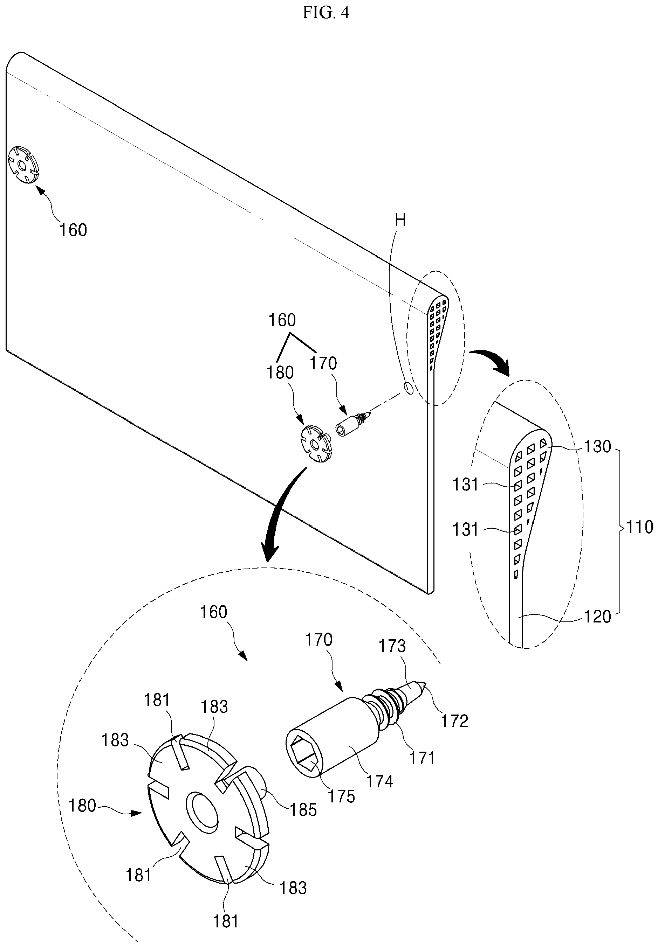

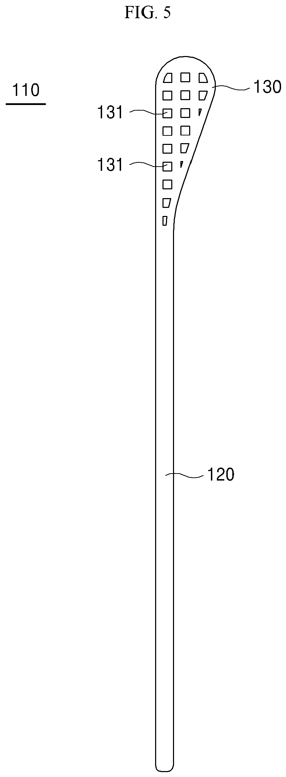

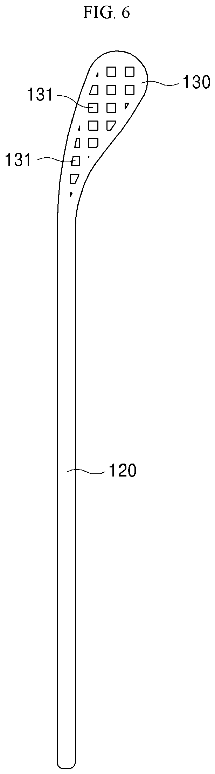

FIG. 2 schematically illustrates a procedure state of a dental membrane set, according to a first embodiment of the present inventive concept. FIG. 3 is an enlarged perspective view of the dental membrane set of FIG. 2. FIG. 4 is a partially exploded view of FIG. 3. FIG. 5 is a side view of a dental membrane. FIG. 6 illustrates a state in which a large area end bending portion of FIG. 5 is bent.

Referring to these drawings, when employing a dental membrane set according to the present embodiment, by adapting a bone defect portion where a bone defect occurs, through an effective structure compared with the related art, the gum epithelium may be prevented from first entering a damaged portion, bone regeneration may also be guided, and furthermore, the failure of guided bone regeneration (GBR) may be reduced.

The scope of right of the present inventive concept having the above effects may be applied not only to a dental membrane 110 of a dental membrane set, but also to a dental membrane set including the dental membrane 110 and a membrane fixing member 160 for fixing the same.

The dental membrane 110 is a substantial structure that prevents the gum epithelium from first entering the damaged portion and also guides bone regeneration, by adapting a bone defect portion where a bone defect occurs.

The dental membrane 110 includes a main plate portion 120 and a large area end bending portion 130.

In the present embodiment, the dental membrane 110 including the main plate portion 120 and the large area end bending portion 130 may be manufactured of titanium or a titanium alloy. However, other materials may be employed, and the scope of right of the present inventive concept is not limited to the above materials.

The main plate portion 120, as illustrated in FIG. 2, substantially serves to prevent the gum epithelium from first entering the damaged portion and also guides bone regeneration, by adapting a bone defect portion where a bone defect occurs. As such, as the main plate portion 120 adapts a bone defect portion, during a procedure, to reduce a sense of foreign material or a sense of reluctance, the main plate portion 120 is made in a thin plate structure.

Although not illustrated in detail in the drawings, a plurality of holes may be further formed in a plate surface of the main plate portion 120. In this state, the size of a hole may vary depending on the position thereof, and also the number of holes may be appropriately selected.

In the present embodiment, the main plate portion 120 is the main plate portion 120 of a rectangular plate-type. Then, it is advantageous to entirely adapt a bone defect portion of a large range such as a rectangle. It is possible to use the main plate portion 120 having a large size by appropriately cutting the same.

While forming one end area of the main plate portion 120, the large area end bending portion 130 is a portion manufactured as one body with the main plate portion 120.

The large area end bending portion 130 has a larger cross-sectional area than that of the main plate portion 120, and is provided to be bendable to one side with respect to the main plate portion 120 as illustrated in FIG. 6 from FIG. 5. In other words, according to the three-dimensional (3D) shape of a bone defect portion, the large area end bending portion 130 may be bent for procedure as illustrated in FIG. 5 or FIG. 6.

The outer surface of the large area end bending portion 130 is processed to be rounded. Accordingly, no injury is caused because there is no sharp end portion.

In addition, as the outer surface, that is, the end portion, is processed to be rounded, when the outer surface is bent, a corresponding portion, that is, a bone defect portion, may be pressed with a relatively strong force. In the present embodiment, the large area end bending portion 130 may be arranged in a bone defect portion to adapt a part of an upper surface in a side surface corner of the bone defect portion as illustrated in FIG. 2. However, the large area end bending portion 130 may be arranged in an area different from the area illustrated in the drawings.

The large area end bending portion 130 may be manufactured in a form in which a cross-sectional area thereof gradually increases from an area contacting the main plate portion 120 toward an end portion thereof.

A plurality of through-holes 131 are formed in the large area end bending portion 130 to cross a direction in which the large area end bending portion 130 is bent. As blood may circulate through the through-holes 131, the effect of bone regeneration may be increased and also a procedure portion may be prevented from festering.

Although, in drawings, the through-holes 131 has a rectangular shape, it is unnecessary that the through-holes 131 each have a rectangular shape. In other words, the through-holes 131 may be formed in various shapes, such as a circular shape, an oval shape, and the like, and in appropriate size and number. Accordingly, the scope of rights of the present inventive concept is not limited to the shapes of the drawings. Furthermore, although the through-holes 131 are illustrated in the drawings, as occasion demands, one through-hole 131 may be formed. All the cases described above are said to be within the scope of right of the present inventive concept.

The membrane fixing member 160 serves to fix the dental membrane 110 to a bone defect portion, as illustrated in FIG. 2.

When the membrane fixing member 160 of the present embodiment to be described below is used, it is advantageous that the dental membrane 110 may be well fixed to a bone defect portion without any lifted part.

When the membrane fixing member 160 according to the present embodiment to be described below is employed instead of general screws, a remarkably improved effect of fixing well the dental membrane 110 is provided.

The membrane fixing member 160 according to the present embodiment includes a bone screw 170 having one side fixed to a bone defect portion, and a bone tack 180 coupled to the bone screw 170 with the dental membrane 110 therebetween and fixing the dental membrane 110.

The bone screw 170 and the bone tack 180 are of a separate type.

The bone screw 170 may be coupled to a bone defect portion, for example, a fixed portion such as an alveolar bone and the like, by being substantially embedded and implanted therein.

The bone screw 170 includes a screw portion 171 fixed to a bone defect portion by a screw method, and a cuff 174 formed coaxially with the screw portion 171, arranged around the screw portion 171, and forming a section having no screw thread.

The screw portion 171 is a portion where a screw thread is formed and that is fixed to a bone defect portion through a screw thread by a screw method. The screw thread may include any of a triangular screw, a rectangular screw, and the like.

Various types of bone screws (not shown) may be used according to the size of the cuff 174, that is, the length of the cuff 174. For example, various types of bone screws (not shown) in which the length of the cuff 174 is 2 mm or 3 mm may be previously prepared for use. This is because the amount of bone differs for each person.

An end portion of the screw portion 171 forming the bone screw 170 forms a sharp tip portion 172 that is sharp. As such, as an end portion of the screw portion 171 is formed to be sharp, the screw portion 171 may be easily and stably fastened by penetrating a bone defect portion. A non-screw portion 173 having no screw thread is formed between the sharp tip portion 172 and the screw portion 171. The non-screw portion 173 is not necessarily formed.

A tool insertion portion 175 having a non-circular shape is formed in an end portion of the cuff 174. The bone screw 170 may be placed and fixed in an alveolar bone and the like of a bone defect portion, via a hole H formed in the dental membrane 110, by inserting a wrench tool and the like into the tool insertion portion 175.

The bone tack 180 serves to fix the dental membrane 110 to the bone screw 170 while coupled to the bone screw 170. Accordingly, the bone tack 180 is formed to have a larger cross-sectional area than the bone screw 170 and presses the dental membrane 110.

The bone tack 180 may include a plurality of unit tacks 183 formed by a cut portion 181. The cut portion 181 may be formed at equal intervals along the circumferential direction.

As such, as the cut portions 181 are formed along the circumferential direction of the bone tack 180, the bone tack 180 may include the unit tacks 183 around the cut portions 181. During fastening of the bone tack 180, the unit tacks 183 may press the main plate portion 120 of the dental membrane 110 of a corresponding portion to fit to the 3D shape of the corresponding portion. Accordingly, it may be prevented that an end portion of the main plate portion 120 around the bone tack 180 is lifted to protrude toward the gum 20 or penetrate the gum 20 to hinder bone regeneration.

A press-fit portion 185 that is press-fitted to the tool insertion portion 175 of the bone screw 170 is provided at a central portion of the unit tacks 183.

Accordingly, after forming the hole H in the dental membrane 110 by using a drill and the like as illustrated in FIG. 4, the bone screw 170 is placed in an alveolar bone by using a wrench tool, and then the bone tack 180 is press-fitted to the bone screw 170 with the dental membrane 110 arranged therebetween, thereby treating the membrane fixing member 160. In this case, it may be advantageous to stably fix the position of the dental membrane 110 without being lifted.

Hereinafter, a series of dental procedure processes using a dental membrane set according to the present embodiment is described.

When treating GBR, first, an operator accurately measures, identify, and recognize the shape of a bone defect portion where a bone defect occurs.

Next, the dental membrane 110 is bent to adapt a bone defect portion. In this state, for example, the task is performed by a method of bending the large area end bending portion 130 to one side with respect to the main plate portion 120 as illustrated in FIG. 6 from FIG. 5.

Next, finally, the dental membrane 110 is coupled to a fixed portion, such as an alveolar bone and the like, by fastening the membrane fixing member 160.

In other words, after forming the hole H in the dental membrane 110 by using a drill and the like, as illustrated in FIG. 4, the bone screw 170 is placed in the alveolar bone by using a wrench tool, and then, the bone tack 180 is press-fitted to the bone screw 170 with the dental membrane 110 arranged therebetween, thereby treating the membrane fixing member 160. When the membrane fixing member 160 is treated by this method, the end portion of the main plate portion 120 around the bone tack 180 may be prevented from being lifted to protrude toward the gum 20 or penetrate the gum 20 to hinder bone regeneration.

According to the present embodiment configured and operating as described above, by adapting a bone defect portion where a bone defect occurs, through an effective structure compared with the related art, the gum epithelium may be prevented from first entering the damaged portion, bone regeneration may be induced, and furthermore, the failure of GBR may be reduced.

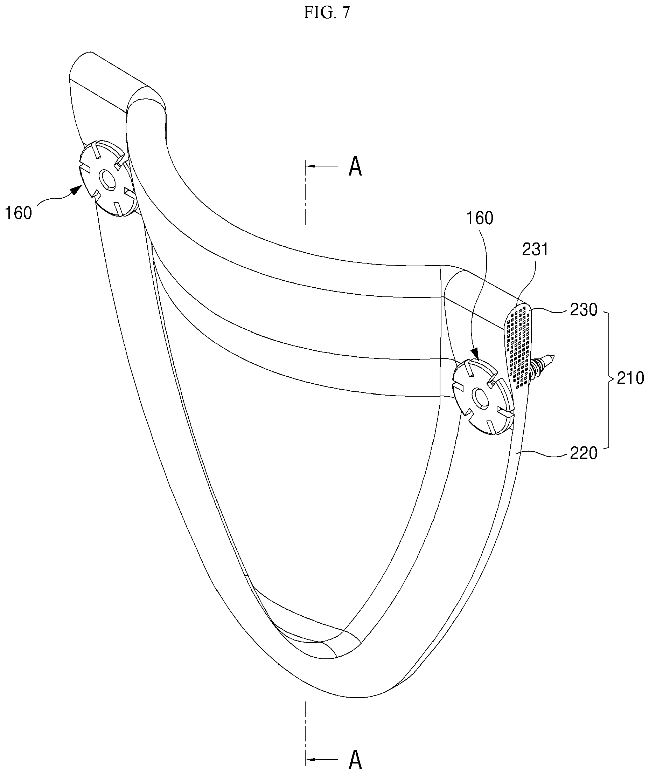

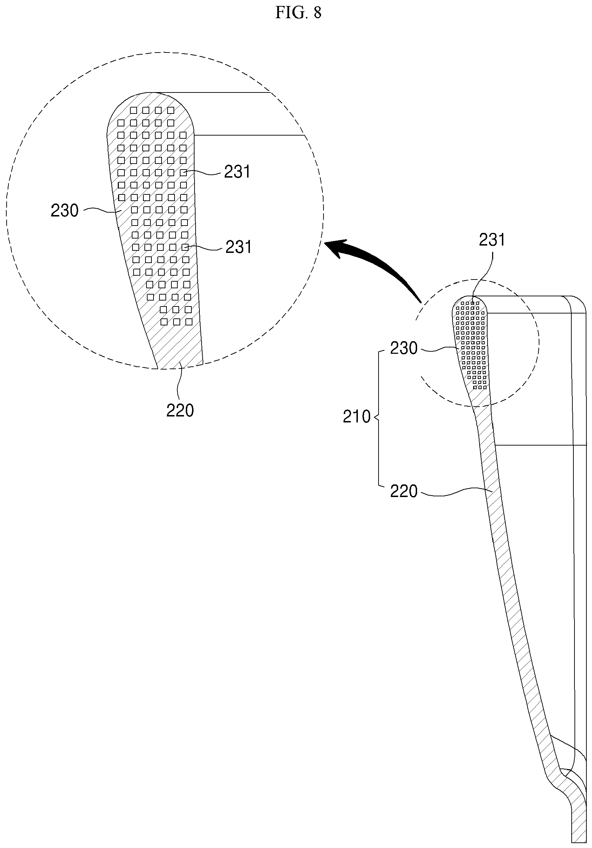

FIG. 7 is a perspective view of a dental membrane set according to a second embodiment of the present inventive concept. FIG. 8 is a cross-sectional view taken along line A-A of FIG. 7.

Referring to these drawings, a dental membrane set according to the present embodiment may also include a dental membrane 210 and the membrane fixing member 160 for fixing the same.

The dental membrane 210 may include a main plate portion 220 and a large area end bending portion 230. A plurality of through-holes 231 for blood circulation may be formed in the large area end bending portion 230.

The main plate portion 220 employed in the present embodiment may have a triangular convex structure to easily adapt an alveolar bone including an area of the tooth root 11 illustrated in FIG. 2. In other words, in the present embodiment, the main plate portion 220 may include the main plate portion 120 of a triangular convex type.

Even when the present embodiment is employed, by adapting a bone defect portion where a bone defect occurs, through an effective structure compared with the related art, the gum epithelium may be prevented from first entering the damaged portion, bone regeneration may be induced, and furthermore, the failure of GBR may be reduced.

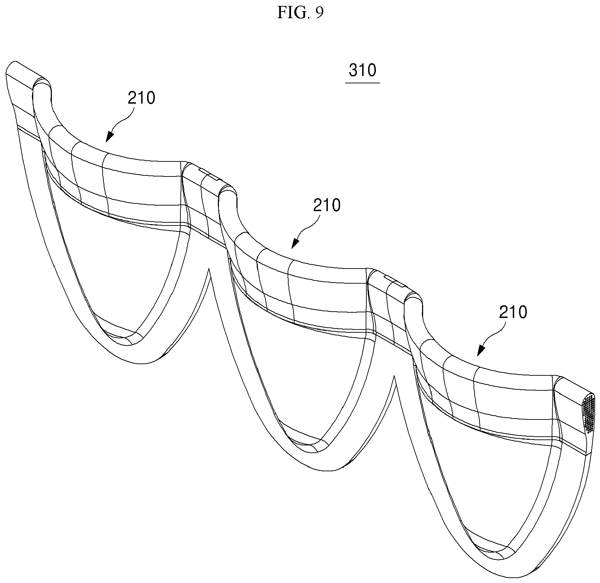

FIG. 9 is a perspective view of a dental membrane set according to a third embodiment of the present inventive concept.

Referring to the drawing, in the case of a dental membrane 310 employed in the present embodiment, the main plate portions 220 illustrated in FIG. 7, that is, the main plate portion 120 of a triangular convex type, are continuously connected in a lateral direction forming one body. In this case, an alveolar bone including an area of neighboring, for example, three, tooth roots 11, not one tooth root 11, may be easily adapted so that the convenience of a procedure may be increased.

Even when the present embodiment is employed, by adapting a bone defect portion where a bone defect occurs, through an effective structure compared with the related art, the gum epithelium may be prevented from first entering the damaged portion, bone regeneration may be induced, and furthermore, the failure of GBR may be reduced.

As such, while the disclosure has been particularly shown and described with reference to preferred embodiments using specific terminologies, the embodiments and terminologies should be considered in descriptive sense only and not for purposes of limitation. Therefore, it will be understood by those of ordinary skill in the art that various changes in form and details may be made therein without departing from the scope of the disclosure as defined by the following claims.

### Industrial Applicability

The present inventive concept is industrially applicable in the dental treatment.

## Claims

1. A dental membrane set comprising:
a dental membrane (110, 210, 310) preventing a gum epithelium from first entering a damaged portion and also guiding bone regeneration, by adapting a bone defect portion where a bone defect occurs; and
a membrane fixing member (160) fixing the dental membrane (110, 210, 310) to the bone defect portion,
**characterised in that** the dental membrane (110, 210, 310) comprises:
a main plate portion (120, 220); and
a large area end bending portion (130, 230) forming an end area in one side of the main plate portion (120, 220), manufactured as one body with the main plate portion (120, 220), having a larger cross-sectional area than the main plate portion (120, 220), and being bendable to one side with respect to the main plate portion (120, 220),
wherein the membrane fixing member (160) comprises:
a bone screw (170) having one side fixed to the bone defect portion; and
a bone tack (180) coupled to the bone screw (170) with the dental membrane (110, 210, 310) therebetween to fix the dental membrane (110, 210, 310),
wherein the bone screw (170) comprises:
a screw portion (171) fixed to the bone defect portion by a screw method; and
a cuff (174) formed coaxially with the screw portion (171), arranged around the screw portion (171), and forming a section having no screw thread.

2. The dental membrane set of claim 1, wherein an end portion of the screw portion (171) has a sharp tip portion (172), a non-screw portion (173) having no screw thread is formed between the sharp tip portion (172) and the screw portion (171), and a tool insertion portion (175) having a non-circular shape is formed in an end portion of the cuff (174).

3. The dental membrane set of claim 2, wherein the bone tack (180) comprises:
a plurality of unit tacks (183) formed by a cut portion (181); and
a press-fit portion (185) provided at a central portion of the plurality of unit tacks (183) and press-fitted to the tool insertion portion (175), and the cut portion (181) is formed along the circumferential direction at equal intervals.

4. The dental membrane set of claim 1, wherein an outer surface of the large area end bending portion (130, 230) is processed to be round, and the large area end bending portion (130, 230) is arranged in the bone defect portion to adapt a part of an upper surface in a side surface corner of the bone defect portion, and the large area end bending portion (130, 230) is manufactured to have a cross-sectional area that gradually increases from an area contacting the main plate portion (120, 220) toward an end portion thereof.

5. The dental membrane set of claim 1, wherein a plurality of through-holes (231) are formed in the large area end bending portion (230) to cross a direction in which the large area end bending portion (230) is bent.

6. The dental membrane set of claim 1, wherein the main plate portion (120, 220) includes a rectangular plate-type main plate portion (120) or a triangular convex-type main plate portion (220).

7. The dental membrane set of claim 6, wherein the triangular convex-type main plate portion (220) includes triangular convex-type main plate portions continuously connected to each other in a lateral direction.

## Patentansprüche

1. Dentalmembransatz, umfassend:
eine Dentalmembran (110, 210, 310), die ein Zahnfleischepithel daran hindert, zuerst in einen geschädigten Abschnitt einzudringen, und die auch die Knochenregeneration führt, indem sie sich einem Knochendefektabschnitt anpasst, wo ein Knochendefekt vorliegt; und
ein Membranfixierelement (160), das die Dentalmembran (110, 210, 310) an dem Knochendefektabschnitt fixiert,
**dadurch gekennzeichnet, dass** die Dentalmembran (110, 210, 310) umfasst:
einen Hauptplattenabschnitt (120, 220); und
einen großflächigen Endbiegeabschnitt (130, 230), der eine Endfläche an einer Seite des Hauptplattenabschnitts (120, 220) bildet und als ein Körper mit dem Hauptplattenabschnitt (120, 220) gefertigt ist, der eine größere Querschnittfläche als der Hauptplattenabschnitt (120, 220) hat und in Bezug zu dem Hauptplattenabschnitt (120, 220) zu einer Seite biegbar ist,
wobei das Membranfixierelement (160) umfasst:
eine Knochenschraube (170), bei der eine Seite an dem Knochendefektabschnitt fixiert ist; und
einen Knochenstift (180), der an die Knochenschraube (170) gekoppelt ist, wobei die Dentalmembran (110, 210, 310) sich dazwischen befindet, um die Dentalmembran (110, 210, 310) zu fixieren,
wobei die Knochenschraube (170) umfasst:
einen Schraubabschnitt (171), der durch ein Schraubverfahren an dem Knochendefektabschnitt fixiert wird; und
eine Manschette (174), die koaxial mit dem Schraubabschnitt (171) gebildet ist, um den Schraubabschnitt (171) herum angeordnet ist und ein Segment ohne Schraubgewinde bildet.

2. Dentalmembransatz nach Anspruch 1, wobei ein Endabschnitt des Schraubabschnitts (171) einen scharfen Spitzenabschnitt (172) aufweist, ein Nicht-Schraubabschnitt (173) ohne Schraubgewinde zwischen dem scharfen Spitzenabschnitt (172) und dem Schraubabschnitt (171) gebildet ist, und ein Werkzeugeinführabschnitt (175) mit einer nicht-kreisförmigen Form in einem Endabschnitt der Manschette (174) gebildet ist.

3. Dentalmembransatz nach Anspruch 2, wobei der Knochenstift (180) umfasst:
eine Vielzahl von Einheitsstiften (183), die durch einen Ausschnittabschnitt (181) gebildet ist; und
einen Presspassabschnitt (185), der an einem zentralen Abschnitt von der Vielzahl der Einheitsstifte (183) bereitgestellt ist und an den Werkzeugeinführabschnitt (175) pressgepasst wird, und wobei der Ausschnittabschnitt (181) entlang der Umfangsrichtung in gleichen Intervallen gebildet ist.

4. Dentalmembransatz nach Anspruch 1, wobei eine Außenoberfläche des großflächigen Endbiegeabschnitts (130, 230) so verarbeitet ist, dass sie rund ist, und der großflächige Endbiegeabschnitt (130, 230) in dem Knochendefektabschnitt angeordnet wird, um sich an einen Teil einer oberen Oberfläche in einer Seitenoberflächenecke des Knochendefektabschnitts anzupassen, und wobei der großflächige Endbiegeabschnitt (130, 230) so gefertigt ist, dass er eine Querschnittfläche hat, die allmählich von einem Bereich, der in Kontakt mit dem Hauptplattenabschnitt (120, 220) ist, in Richtung eines Endabschnitts davon zunimmt.

5. Dentalmembransatz nach Anspruch 1, wobei eine Vielzahl von Durchgangslöchern (231) in dem großflächigen Endbiegeabschnitt (230) gebildet ist, so dass sie eine Richtung queren, in die der großflächige Endbiegeabschnitt (230) gebogen ist.

6. Dentalmembransatz nach Anspruch 1, wobei der Hauptplattenabschnitt (120, 220) einen Hauptplattenabschnitt (120) vom Typ rechteckige Platte oder einen Hauptplattenabschnitt (220) vom dreieckig-konvexen Typ einschließt.

7. Dentalmembransatz nach Anspruch 6, wobei der Hauptplattenabschnitt (220) vom dreieckig-konvexen Typ Hauptplattenabschnitte vom dreieckig-konvexen Typ einschließt, die in einer lateralen Richtung kontinuierlich miteinander verbunden sind.

## Revendications

1. Kit de membrane dentaire comprenant :
une membrane dentaire (110, 210, 310) empêchant un épithélium de la gencive de pénétrer d'abord dans une partie endommagée et guidant aussi la régénération osseuse, en adaptant une partie de déficit osseux là où un déficit osseux se produit ; et
un membre de fixation de membrane (160) fixant la membrane dentaire (110, 210, 310) à la partie de déficit osseux,
**caractérisé en ce que** la membrane dentaire (110, 210, 310) comprend :
une partie de plaque principale (120, 220) ; et
une partie de courbure de grande extrémité de zone (130, 230) formant une zone d'extrémité dans un côté de la partie de plaque principale (120, 220), fabriquée en tant qu'un seul corps avec la partie de plaque principale (120, 220), ayant une aire transversale plus grande que la partie de plaque principale (120, 220), et pouvant être courbée vers un côté
par rapport à la partie de plaque principale (120, 220),
dans lequel le membre de fixation de membrane (160) comprend :
une vis à os (170) ayant un côté fixé sur la partie de déficit osseux ; et
un clou osseux (180) couplé à la vis à os (170) avec la membrane dentaire (110, 210, 310) entre pour fixer la membrane dentaire (110, 210, 310),
dans lequel la vis à os (170) comprend :
une partie de vis (171) fixée à la partie de déficit osseux par un procédé de vis ; et
un procédé par vis, et
un manchon (174) formé coaxialement avec la partie de vis (171), agencé autour de la partie de vis (171) et formant une section n'ayant pas de filet de vis.

2. Kit de membrane dentaire selon la revendication 1, dans lequel une partie d'extrémité de la partie de vis (171) présente une partie d'embout pointu (172), une partie de non-vis (173) n'ayant pas de filet de vis est formée entre la partie d'embout pointu (172) et la partie de vis (171), et une partie d'insertion d'outil (175) ayant une forme non-circulaire est formée dans une partie d'extrémité du manchon (174).

3. Kit de membrane dentaire selon la revendication 2, dans lequel le clou osseux (180) comprend :
une pluralité de clous unitaires (183) formés par une partie découpée (181) : et
une partie d'insertion par force (185) prévue sur une partie centrale de la pluralité de clous unitaires (183) et insérée par force sur la partie d'insertion d'outil (175), et la partie découpée (181) est formée le long de la direction circonférentielle à intervalles réguliers.

4. Kit de membrane dentaire selon la revendication 1, dans lequel une surface extérieure de la partie de courbure de grande extrémité de zone (130, 230) est usinée pour être ronde, et la partie de courbure de grande extrémité de zone (130, 230) est agencée dans la partie de déficit osseux pour adapter une partie d'une surface supérieure dans un coin de surface latéral de la partie de déficit osseux, et la partie de courbure de grande extrémité de zone (130, 230) est fabriquée pour avoir une aire transversale qui augmente graduellement d'une zone contractant la partie de plaque principale (120, 220) vers une partie d'extrémité de celle-ci.

5. Kit de membrane dentaire selon la revendication 1, dans lequel une pluralité de trous traversants (231) sont formés dans la partie de courbure de grande extrémité de zone (230) pour croiser une direction dans laquelle la partie de courbure de grande extrémité de zone (230) est courbée.

6. Kit de membrane dentaire selon la revendication 1, dans lequel la partie de plaque principale (120, 220) inclut une partie de plaque principale de type plaque rectangulaire (120) ou une partie de plaque principale de type convexe triangulaire (220).

7. Kit de membrane dentaire selon la revendication 6, dans lequel la partie de plaque principale de type convexe triangulaire (220) inclut des parties de plaque principale de type convexe triangulaire reliées en continu l'une à l'autre dans une direction latérale.
